# EUROPEAN PATENT APPLICATION

(11) **EP 4 748 801 A1**
(43) Date of publication of application: **27.05.2026**
(21) Application number: 24842495.4
(22) Date of filing: 15.07.2024
(51) Int. Cl.: C02F 1/78

(54) **LIQUID OZONIZATION DEVICE**

(30) Priority: 17.07.2023 ES 202331300 U
(71) Applicant: Schwartz Tapia, Adriana del Carmen, 28035 Madrid (ES)
(72) Inventor: Schwartz Tapia, Adriana del Carmen, 28035 Madrid (ES)
(74) Representative: Munoz Garcia, Antonio
(86) International application number: PCT/ES2024/070448
(87) International publication number: WO 2025/017235

(57) **Abstract**

LIQUID OZONIZATION DEVICE comprising: a transparent container (1) with graduation marks (11); a glass coil (2) interior to the container (1) that spans from its lower to its upper end; an orifice-equipped micro-bubbling generating plate (3) at the bottom of the container (1), connected to the coil (2); an ozone inlet port (5) that connects the coil (2) with a siliconized cannula (12) to an ozone generator; a stopcock (4) in the ozone inlet port (5); an ozone outlet port (6) that connects the container (1) with another siliconized cannula (13) to an ozone destructor; a liquid inlet port (7) to the container (1); and an ozonized liquid outlet port (8).

## Description

### OBJECT OF THE INVENTION

The invention, as expressed in the title of this specification, refers to a liquid ozonization device that contributes advantages and characteristics to the function for which it is intended, which are described in detail below, and which represent an improvement of the current state of the art.

More specifically, the object of the invention focuses on a device for the treatment of liquids by means of ozone, mainly water, more specifically a device comprising a reservoir with mixing means under ozone micro-bubbling, which, in addition to achieving a bubble size with a diameter between 10 and 50 µm that decrease in size to finally disappear under water, advantageously allowing them to remain in the water for a long time and act as a battery that continuously supplies ozone, presents a practical, reusable, lightweight and easy-to-handle design, compatible with all devices on the market related to ozone therapies.

### FIELD OF APPLICATION OF THE INVENTION

The field of application of the present invention falls within the industrial sector dedicated to the manufacture of ozonization apparatuses and devices, focusing particularly on those applicable for water ozonization.

### BACKGROUND OF THE INVENTION

As is known, water ozonization is a process whereby ozone is used to disinfect and purify water. Ozone (Os) is a highly reactive form of oxygen and a powerful oxidant. ⁵It is commonly used in water treatment plants and purification systems to eliminate bacteria, viruses, fungi, algae, and other contaminants present in the water.

Water ozonization offers several important benefits. These are some of the main uses and advantages:
- Controlling fungi, microorganisms, and algae in plants.
- Controlling the growth of fungi and microorganisms by washing udders of milk-extracting animals with these solutions.
- Increasing the shelf life of foods, including citrus fruits, vegetables, fruits, meats, fish, eggs, and dairy products.
- Improving the quality of potable drinking water. Ozone is at the same time a metal chelator.
- Using water for mouthwashes and wound cleaning treatments.

For the application of ozone in water, ozonizers or devices to generate ozone and dissolve it in water are known. Essentially, said devices comprise the following elements:
- An ozone generator, which is responsible for producing ozone through different methods, and which usually has electrodes or special plates that facilitate ozone production.
- A contact or mixing tube, where, once generated, the ozone is introduced into said tube or reactor where it mixes with the water, being specially provided to guarantee high efficiency of ozone transfer to the water, allowing for adequate dissolution.
- And an injection or diffusion system, to ensure effective mixing of the ozone with the water, which may consist of a Venturi injector or a diffuser stone that is placed in the contact tube. These systems create a water flow that suctions or disperses the ozone, facilitating its dissolution in the water.

In general, different contact tube designs exist to maximize ozone dissolution and mixing with the water. Some systems use a contact tube with a spiral or coil design, while others employ a reactor design with multiple stages or chambers.

When ozone is introduced into the contact tube, the following processes occur:
- Ozone, which is a gas, dissolves in the water upon coming into contact with it. The dissolution of ozone is more effective when there is a large contact surface between the gas and the water.
- During dissolution, ozone diffuses from the gas phase to the liquid phase. This process is due to a concentration difference between the ozone in the gas and the ozone dissolved in the water.
- Once dissolved in the water, ozone can react with various substances present, such as microorganisms, organic or inorganic compounds. These reactions help in disinfection and the elimination of contaminants.

As stated, some systems incorporate Venturi injectors or diffuser stones to create fine ozone bubbles in the water. These bubbles increase the contact surface between the ozone and the water, favoring dissolution and the transfer of ozone to the liquid.

The size of the ozone bubbles can vary depending on the characteristics of the system and the design of the contact tube. In general, ozone bubbles usually have a small diameter, in the range of micrometers to millimeters, which allows for a greater surface area for ozone transfer to the water and better efficiency in the ozonization process.

Therefore, the objective of the present invention is the development of an ozonizer device, specifically a mixing tube that achieves a minimum size of ozone bubbles, to favor their permanence in the water and increase the effectiveness of the device, constituting, at the same time, a reusable, lightweight, and easy-to-handle apparatus, which, furthermore, is compatible with existing devices on the market related to ozone therapies or treatments.

On the other hand, and as a reference to the current state of the art, it should be noted that, although as stated other ozonizer devices are known, at least on the part of the applicant, the existence of any that presents technical and structural characteristics equal or similar to those claimed herein is unknown.

### EXPLANATION OF THE INVENTION

The liquid ozonization device proposed by the invention represents an improvement over what is already known, with the characterizing details that make it possible and distinguish it being conveniently gathered in the final claims accompanying this description.

What the invention proposes, as previously noted, is a device for ozonizing water or other liquids under micro-bubbling, more specifically a device or contact tube comprising a reservoir with means for mixing and generating ozone bubbles, with a practical reusable, lightweight, and easy-to-handle design compatible with all devices on the market related to ozone therapies.

Specifically, the micro-bubbles generated by the device object of the invention are small bubbles with a diameter between 10 and 50 µm, which decrease in size to finally disappear under the water.

The size of a bubble is the first determining characteristic for understanding its properties, since a size distribution on the micro/nanometric scale is associated with better stability and mass transfer, significantly influencing its behavior and other physicochemical and electrical characteristics within a liquid.

Larger bubbles tend to emerge showing a greater buoyancy force, whereas smaller bubbles will remain in the liquid medium with greater ease and duration with a random movement pattern or Brownian motion.

The micro-bubbles remain in the water for a long time and act as a battery that continuously supplies ozone to the water. As the ozone is consumed, they diffuse more quantity, maintaining the level of ozone dissolved in the water. The micro-bubbles do not go to the surface, but rather are distributed homogeneously within the water mass, guaranteeing its sterilization.

The antimicrobial action of ozone micro-bubbles is exerted from the free radicals generated at the moment of cavitation of the micro-bubbles; likewise, the pH of the solutions where they are found significantly affects the amount of free radicals generated, where a lower pH will increase the number of hydroxyl group free radicals.

Furthermore, the inhibition of bubble coalescence, that is, of the fusion process to form larger bubbles and therefore the result of the smaller bubble size, leads to an increase in the mass of ozone gas transferred to the water or saline solution.

Micro-bubbles, unlike other larger bubbles, possess unique characteristics such as their surface charge which confers on them a half-life of up to 6 months under in vitro conditions.

For all of this, the water ozonization device essentially comprises the following elements:
- A glass container with a configuration in the form of a graduated vertical tube, preferably with a capacity of 250ml.
- A glass coil incorporated inside the container, such that it runs internally from one end to the other.
- A micro-bubbling glass plate incorporated at the bottom of the container, connected to the lower end of the coil, so that the generated bubbles emerge through it at said bottom of the container.
- An ozone inlet port or entrance, at the upper end of the coil, shaped in the form of a duct for connecting the device to a siliconized cannula which, in turn, connects to an ozone generator.
- A stopcock inserted in the duct of the ozone inlet port, at the upper end of the coil, which allows controlling the entry of ozone thereto.
- An ozone outlet port or connection of the device container to a second siliconized cannula which, in this case, connects to an external ozone destructor or ozone catalyst, preferably to convert the excess ozone not used in the ozonization process, that is, the bubbles that have risen to the upper part of the liquid inside the container, into molecular oxygen (O₂), which is a more stable and safe form.
- A liquid inlet port, normally water or saline solution, to the device container situated at the upper end of said container.
- And an ozonized water outlet port with the device situated at the lower end of the container and which connects to an outlet cannula.

Finally, in a preferred embodiment option, the device comprises a support to be able to place the container, with the set of elements it comprises coupled thereto, on some appropriate element provided for that purpose so that it is held in a vertical position and can act correctly. Preferably, said support is formed by an elongated piece, preferably a metal strip, with a hanging hole that is integrally joined to the glass container.

### DESCRIPTION OF THE DRAWINGS

To complement the description being made and with the object of aiding a better understanding of the characteristics of the invention, attached to this descriptive specification, as an integral part thereof, is a set of drawings in which, for illustrative and non-limiting purposes, the following has been represented:
Figure number 1.- Shows a front perspective view of an embodiment example of the liquid ozonization device object of the invention, appreciating the main parts and elements it comprises as well as the configuration and arrangement thereof.

### PREFERRED EMBODIMENT OF THE INVENTION

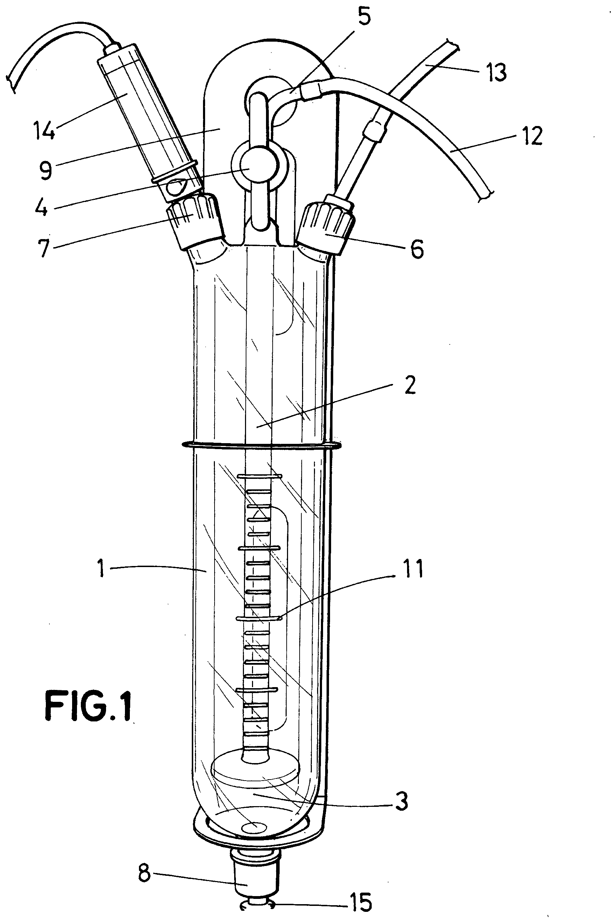

In view of the described figure 1 and only figure, and in accordance with the numbering adopted therein, a non-limiting embodiment example of the ozonization device of the invention can be observed, which comprises what is indicated and described in detail below.

Thus, as appreciated in said figure, the device (10) of the invention is of the type comprising a liquid container (1) with a coil (2) with perforations connected to an ozone generator to generate bubbles that mix with the liquid. And, based on said already known configuration, it is essentially distinguished by comprising:
- As a liquid container (1), a transparent glass container (1), which is of a configuration in the form of a closed vertical tube, preferably with a capacity of 250ml, and is provided with graduation marks (11) to know the volume of liquid it contains.
- As a coil (2), a coil (2) also made of glass that is incorporated inside the container (1), and which spans practically its entire interior from its lower to its upper end.
- An orifice-equipped glass plate for the generation of micro-bubbling (3) incorporated at the bottom of the container (1), connected to the coil (2), so that the ozone emerges through it in the form of micro-bubbles at said bottom of the container (1).
- An ozone inlet port (5) which, shaped in the form of a duct, connects one end of the coil (2), at the upper end of the container (1), to a first siliconized cannula (12) which, in turn, connects to an ozone generator (not represented).
- A stopcock (4) inserted in the previously mentioned duct of the ozone inlet port (5), at the upper end of the container (1), which allows opening or closing the passage of ozone to the coil (2).
- An ozone outlet port (6), also in the form of a duct, which connects the upper part of the container (1) with a second siliconized cannula (13) which, in turn, connects to an external ozone destructor or catalyst (not represented).
- A liquid inlet port (7) to the container (1), situated at the upper end of said container (1), which is introduced through a conduit provided, preferably, with a stop valve (14).
- And an ozonized liquid outlet port (8) situated at the lower end of the container (1) and which, in turn, connects to an outlet cannula (15), being able to incorporate a stop valve (not shown).

Preferably, the perforations of the micro-bubbling plate (3) have a diameter of between 10 and 50 µm such that they generate ozone bubbles of said dimensions. Preferably, said plate (3) is a circular plate that is perforated perimetrally.

Finally, in a preferred embodiment option, the device comprises a support (9) to be able to place the container (1) with all its elements held in a vertical position. Preferably, said support (9) is an elongated piece, preferably a metal strip, with a hanging hole that is integrally joined, for example glued with some type of glue, to the glass container (1) on its external rear part, that is, on the part opposite to the one incorporating the graduation marks (11).

Having sufficiently described the nature of the present invention, as well as the manner of putting it into practice, it is not considered necessary to make its explanation more extensive so that any expert in the matter may understand its scope and the advantages derived from it, stating that, within its essentiality, it may be put into practice in other embodiments that differ in detail from that indicated by way of example, and to which the protection claimed shall equally extend provided that its fundamental principle is not altered, changed or modified.

## Claims

1. LIQUID OZONIZATION DEVICE which, comprising a liquid container (1) with a coil (2) with perforations connected to an ozone generator to generate bubbles that mix with the liquid, is **characterized in that** it comprises:
• a transparent glass container (1) of a configuration in the form of a closed vertical tube and provided with graduation marks (11);
• a glass coil (2) incorporated in the container (1), and which spans practically its entire interior from its lower to its upper end;
• an orifice-equipped glass plate for the generation of micro-bubbling (3) incorporated at the bottom of the container (1), connected to the coil (2), such that the ozone emerges through it in the form of micro-bubbles at said bottom of the container (1);
• an ozone inlet port (5) that connects one end of the coil (2), at the upper end of the container (1), to a first siliconized cannula (12) which, in turn, connects to an ozone generator;
• a stopcock (4) inserted in the duct of the ozone inlet port (5), at the upper end of the container (1), to open or close the passage of ozone to the coil (2);
• an ozone outlet port (6) that connects the upper end of the container (1) with a second siliconized cannula (13) which, in turn, connects to an external ozone destructor or catalyst;
• a liquid inlet port (7) to the container (1), situated at the upper end of said container (1); and
• an ozonized liquid outlet port (8) situated at the lower end of the container (1).

2. LIQUID OZONIZATION DEVICE, according to claim 1, **characterized in that** the perforations of the micro-bubbling plate (3) have a diameter of between 10 and 50 µm, such that they generate ozone bubbles of said dimensions.

3. LIQUID OZONIZATION DEVICE, according to claim 2, **characterized in that** said micro-bubbling plate (3) is a circular plate that is perforated perimetrally.

4. LIQUID OZONIZATION DEVICE, according to any of the preceding claims, **characterized in that** the transparent glass container (1) of a configuration in the form of a closed vertical tube has a capacity of 250ml.

5. LIQUID OZONIZATION DEVICE, according to any of the preceding claims, **characterized in that** it comprises a support (9) to place the container (2) with all its elements held in a vertical position.

6. LIQUID OZONIZATION DEVICE, according to claim 5, **characterized in that** the support (9) is an elongated, metallic piece, with a hanging hole that is integrally joined to the glass container (1) on its external rear part.
